# EUROPEAN PATENT APPLICATION

(11) **EP 4 740 964 A1**
(43) Date of publication of application: **13.05.2026**
(21) Application number: 24835231.2
(22) Date of filing: 19.06.2024
(51) Int. Cl.: A61K 47/42, C07K 19/00, C12N 15/62

(54) **NANO DELIVERY SYSTEM, PREPARATION METHOD THEREFOR AND USE THEREOF**

(30) Priority: 03.07.2023 CN 202310808097
(71) Applicant: Chen, Haiyan, Guangzhou, Guangdong 510080 (CN); Guangzhou Nanopac Biotechnology Ltd, Guangzhou, Guangdong 510799 (CN)
(72) Inventor: CHEN, Haiyan, Guangzhou, Guangdong 510080 (CN)
(74) Representative: Høiberg P/S
(86) International application number: PCT/CN2024/100135
(87) International publication number: WO 2025/007748

(57) **Abstract**

Provided are a nano-delivery system, comprising ferritin, a targeting protein and an active molecule, a preparation method thereof, and a use thereof. This can provide targeted delivery system and a targeted therapeutic method that are safe, stable, low toxic, highly targeted, multispecific, and convenient to prepare.

## Description

### FIELD OF THE INVENTION

This invention belongs to the field of drug delivery systems for disease diagnosis and treatment.

### BACKGROUND OF THE INVENTION

Small molecule drugs, such as tumor chemotherapeutic agents, are widely used in disease treatment. However, the clinical application of such drugs is limited by their aqueous solubility, distribution in tissues, specificity for cells and toxicity (Z.G. Chen, Small-molecule delivery by nanoparticles for anticancer therapy. Trends Mol Med 16, 594-602 (2010); L. M. Ickenstein and P. Garidel, Lipid-based nanoparticle formulations for small molecules and RNA drugs. Expert Opin Drug Deliv 16, 1205-1226 (2019)).

The high expression of specific molecules on the surface of certain cells, e.g. tumor cells, enables antibody-based therapies, which achieve precise cell-targeting by binding to these specific molecules. Such antibody-mediated targeting therapies include CAR T-cell therapies, antibody-drug conjugate (ADC) therapies, bispecific antibody therapies, etc. However, currently developed CAR-T cell therapies only target hematological tumors, exhibit significant side effects, and are expensive, time-consuming and only applicable to specific subjects. The ADC therapies have cytotoxic drugs exposed to normal cells or tissues, resulting in insufficient stability and significant side effects. The bispecific antibody therapies may cause adverse reactions and have issues such as low stability. Therefore, there is still a need for safe, convenient, stable, low toxic, high targeting specificity, and commonly used targeted delivery systems and targeting therapies.

### SUMMARY OF THE INVENTION

This disclosure addresses the need for the targeted delivery systems and targeting therapies that are safe, stable, low toxic, high targeting specificity, multispecific, and convenient to prepare.

The disclosure is based on a surprising finding by the inventors that conjugating antibodies to ferritin nanoparticles using Sortase A and encapsulating active molecules, such as small molecule therapeutics, within the nanoparticles may conveniently generate a safe, highly stable, low-toxic, precisely targeting, and commonly used targeted delivery system. Since a ferritin nanoparticle is composed of 24 ferritin monomers (or referred to as subunits), the nanoparticle may theoretically conjugate 24 antibody molecules using Sortase A, enabling the enhanced antibody affinity. Alternatively, a plurality of different antibodies may be conjugated, thereby enabling targeting of multiple targets and achieving multispecificity.

Earlier studies have utilized human ferritin to target human transferrin receptor 1 (TfR1). Human TfR1 is highly expressed in tumor cells, enabling targeted delivery of drugs. However, human TfR1 is also widely expressed in healthy tissues in human, such as bone marrow, lung, colon, and liver, to transport irons into cells. Therefore, the use of human ferritin may lead to undesirable risks of accumulating drugs in healthy tissues and of autoimmunity. The inventors have further surprisingly found that modifying the ferritin-encoding gene derived from *Helicobacter pylori* and expressing the modified gene in mammalian cells (e.g., CHO cells) can address these problems. Furthermore, the ferritin obtained in this way exhibits a conformation more closely resembling its native status, and simultaneously has a high yield ( more than 80 mg/L cells ).

In one aspect, herein provided is a nano-delivery system, comprising a ferritin nanoparticle, a targeting protein, and an active molecule, wherein the targeting protein molecule is conjugated onto the surface of the ferritin nanoparticle through the linker set forth in SEQ ID NO. 19 (LPXTGGGG, where X can be any amino acid), and the active molecule is encapsulated within the ferritin nanoparticle.

In some embodiments, the amino acid sequence of the ferritin in the nano-delivery system is set forth in SEQ ID NO. 11.

In the nano-delivery system described herein, the targeting protein may be any protein with the ability to target, e.g., with the ability to target specific cells, such as tumor cells. For example, the targeting protein is an antibody molecule e.g., IgG antibody, or an antigen-binding fragment thereof.

In the nano-delivery system described herein, the active molecule is any molecule desired for targeted delivery that can be encapsulated in the ferritin nanoparticle. For example, the active molecule is any suitable therapeutic agent, such as a small molecule drug, e.g., an anti-tumor drug, as long as it can be encapsulated in the ferritin nanoparticle as used herein.

In the nano-delivery system as described herein, the ferritin nanoparticle and the targeting protein are conjugated together using Sortase A, thereby forming a linker set forth in SEQ ID NO. 19 (LPXTGGGG, wherein X is any amino acid) between them ( the conjugate is in the form of N-terminus - targeting molecule - linker - ferritin - C-terminus).

In another aspect, herein provided is a method for preparing a nano-delivery system, comprising:
1) encapsulating an active molecule within a ferritin nanoparticle; and
2) conjugating the ferritin nanoparticle to a targeting protein using Sortase A.

As used herein, steps 1) and 2) are performed in one reaction or in two separate reactions.

In some embodiments, the method further comprises a step of providing a ferritin with the amino acid sequence set forth in SEQ ID NO. 20 (GGGG) linked to the N-terminus, and a targeting protein with the amino acid sequence set forth in SEQ ID NO. 21 (LPXTGG) linked to the C-terminus.

In some embodiments, the amino acid sequence of the ferritin in the nano-delivery system is set forth in SEQ ID NO. 11.

In the method described herein, the targeting protein for the nano-delivery system is any protein with the ability to target, e.g., the ability to target specific cells, such as tumor cells. For example, the targeting protein is an antibody molecule, e.g., IgG antibody, or an antigen-binding fragment thereof. In the case of an antibody molecule, the amino acid sequence set forth in SEQ ID NO. 21 (LPXTGG) may be added to the C-terminus of a heavy chain.

In the method described herein, the active molecule is any molecule desired for targeted delivery that can be encapsulated in the ferritin nanoparticle. For example, the active molecule is a suitable therapeutic agent, such as a small molecule drug, e.g., an anti-tumor drug, as long as they can be encapsulated by the ferritin nanoparticle as used herein.

In a further aspect, herein provided is a modified ferritin protein, of which the sequence is set forth in SEQ ID NO. 11.

In a further aspect, herein provided is a polynucleotide encoding a modified ferritin having an amino acid sequence set forth in SEQ ID NO. 11. For example, the polynucleotide has a nucleotide sequence set forth in SEQ ID NO. 2.

In a further aspect, herein provided is use of a modified ferritin or a polynucleotide encoding the modified ferritin as described herein in the preparation of a nano-delivery system.

In a further aspect, herein provided is a nano-delivery system as described herein for use in treating cancer.

In a further aspect, herein provided is a method for treating cancer in a subject, comprising administering to the subject a nano-delivery system as described herein .

In some embodiments, by using a tumor-targeting antibody, an anti-tumor small molecule drug and a nanoparticle, an antibody-ferritin nanoparticle with the function of antibody-mediated targeted delivery of the small molecule drug is produced, which can be used as an anti-tumor nanoparticle drug with precise targeting, high stability and low toxicity.

In some embodiments, a targeting antibody for B-cell non-Hodgkin's lymphoma or lymphocytic leukemia (e.g. CD19 IgG, CD19 Fab, CD20 IgG, or CD22 IgG) is conjugated onto the surface of a nanoparticle in which a small molecule drug against the disease is encapsulated via Sortase A. Said antibody precisely targets the diseased cell and mediates the delivery of the small molecule drug to the cell, thereby reducing toxicity to normal cells and achieving enhanced stability. Hereby a targeted therapeutic agent for B-cell non-Hodgkin's lymphoma or lymphocytic leukemia, with high targeting precision, high stability, and low toxicity, is provided.

### BRIEF DESCRIPTION OF THE DRAWINGS

The following embodiments will be described in conjunction with the drawings, so that the aforementioned and other aspects and advantages of the present disclosure will become apparent and readily understood.
Figure 1 shows as an illustration the passive permeation of ferritin proteins at high temperatures and their dissociation-reassembly procedure under strong acidic and alkaline conditions.
Figure 2 shows as an illustration a flowchart of preparing a nano-delivery system in which the anti-tumor drug, doxorubicin (DOX), is encapsulated. Under specific temperature or pH conditions, the small molecule drug is encapsulated within a ferritin nanoparticle. Then, an IgG antibody or an antigen-binding fragment (Fab) is conjugated to the ferritin nanoparticle by Sortase A, forming nanoparticle for targeted delivery of the drug.

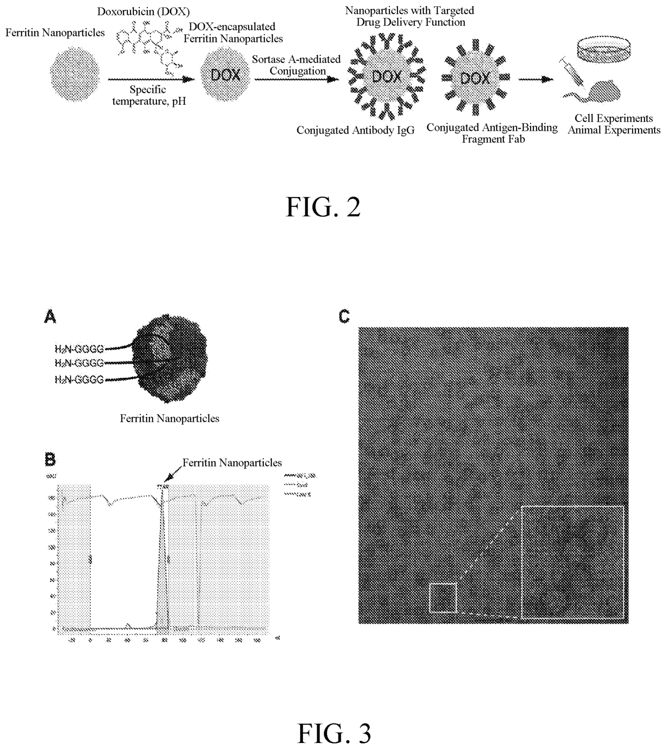

Figure 3 shows as an illustration the preparation of ferritin nanoparticles mentioned herein. (A) Schematic illustration of ferritin nanoparticles. Each nanoparticle is composed of 24 copies of ferritin as subunits, with the N-terminus of each ferritin protein linked to the amino acid sequence set forth in SEQ ID NO. 20 (GGGG) useful in the conjugation to the targeting protein via Sortase A. (B) Separation of the ferritin nanoparticles by HPLC. The peak indicated by the arrow demonstrates the high purity of the ferritin nanoparticles. (C) The morphology of the ferritin nanoparticles observed using negative-stain electron microscopy.

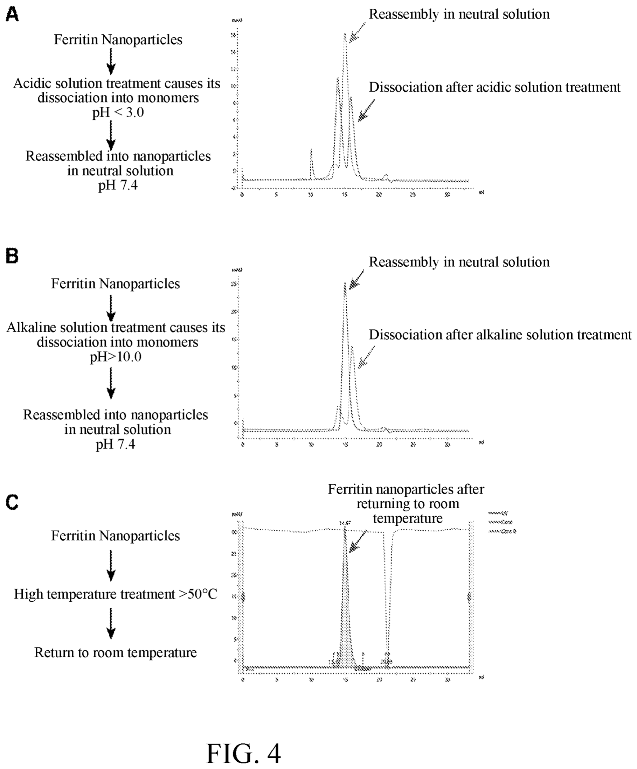

Figure 4 shows that the ferritin nanoparticles mentioned herein may be dissociated or become loose under strongly acidic, strongly alkaline, or high-temperature conditions, and reassemble or revert back to their normal states under neutral pH or ambient temperature conditions, suggesting their potential to encapsulate small molecule drugs. (A) HPLC analysis shows that ferritin nanoparticles dissociate into ferritin monomers after treatment with an acidic solution having a pH value of less than 3.0. The solution is then replaced with a PBS solution having a pH value of 7.4, and the ferritin monomers reassemble into nanoparticles. (B) HPLC analysis shows that the ferritin nanoparticles dissociate into ferritin monomers after treatment with a alkaline solution having a pH value of more than 10.0. The solution is then replaced with neutral PBS having a pH value of7.4, and the ferritin monomers reassemble into the nanoparticles. (C) HPLC analysis shows that the ferritin nanoparticles exist in a loosely bound state with enlarged pore size after heat treatment at temperatures above 50°C, thereby allowing the entry of drugs into the nanoparticles, and then the ferritin nanoparticles can revert back to their original particle states when the temperature is restored to ambient temperature (20-25°C).
Figure 5 shows as an illustration the encapsulation of DOX by ferritin nanoparticles (referred to as Ferritin-Dox). (A) Schematic illustration of ferritin nanoparticles. Each nanoparticle is composed of 24 copies of ferritin as subunits. (B) Separation of the ferritin nanoparticles by HPLC. The peak indicated by the arrow demonstrates the high purity of the ferritin nanoparticles. (C) The morphology of the DOX-encapsulated ferritin nanoparticles bynegative-stain electron microscopy.

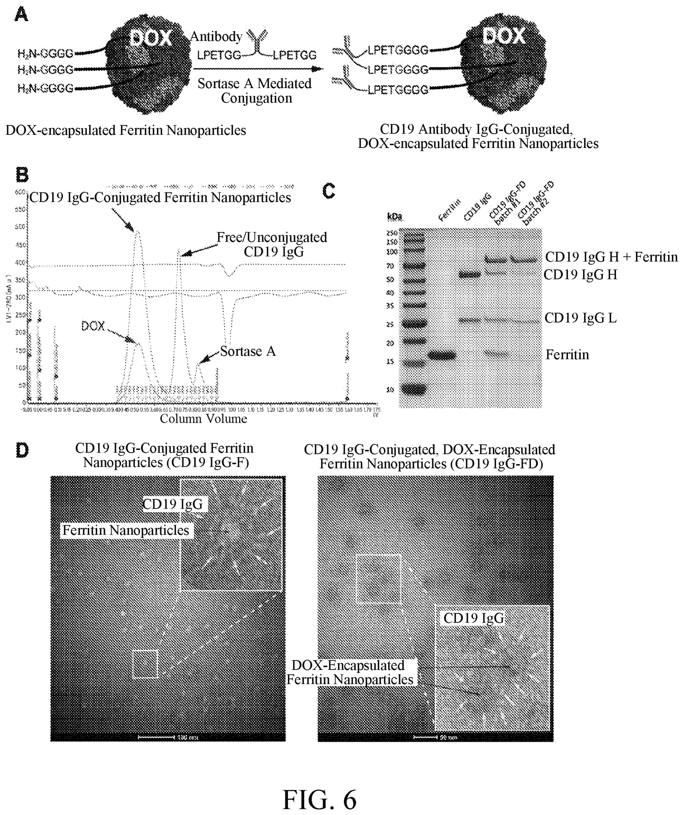

Figure 6 shows as an illustration the ferritin nanoparticle (referred to as F) with encapsulated DOX (referred to as D), which is conjugated with CD19 IgG antibodies (the conjugate is referred to as CD19 IgG-FD). (A) Schematic illustration of the IgG antibody-conjugated, DOX-encapsulated ferritin nanoparticles. IgG antibody is conjugated to the ferritin subunits by using Sortase A, with a maximum of 24 IgG antibodies bound to the surface of each ferritin nanoparticle. (B) Analysis and purification by HPLC. (C) SDS-PAGE analysis of the purified product, CD19 IgG-FD. (D) The morphology of the nanoparticle before and after encapsulating DOX by negative-stain electron microscopy (left: CD19 IgG-F, before the encapsulation; right: CD19 IgG-FD, after the encapsulation).

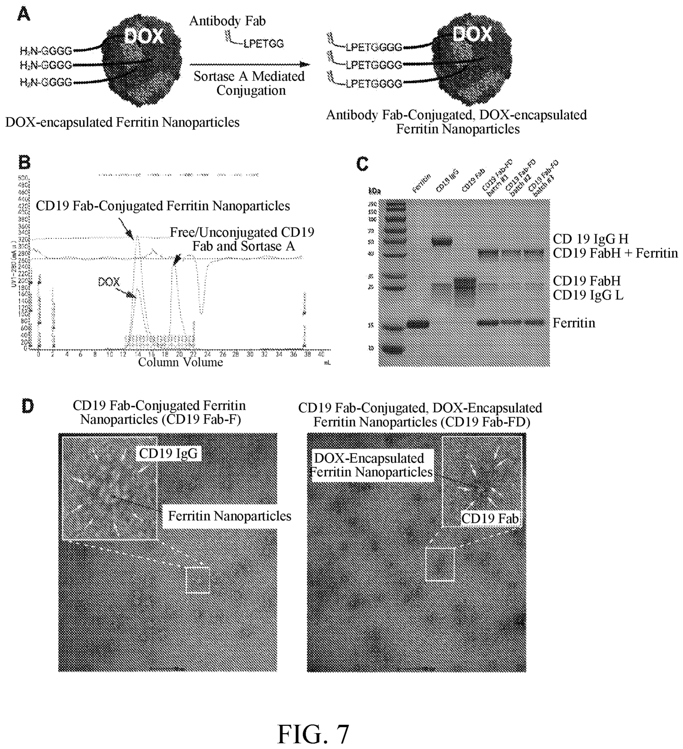

Figure 7 shows as an illustration the CD19 antibody antigen-binding fragments (Fab)-conjugated, DOX-encapsulated ferritin nanoparticles (referred to as CD19 Fab-FD). (A) Schematic illustration of the Fab fragment-conjugated, DOX-encapsulated ferritin nanoparticles. Fab fragment is conjugated to the ferritin subunits by using Sortase A, with a maximum of 24 IgG antibodies bound to the surface of each ferritin nanoparticle. (B) Analysis and purification by HPLC. (C) SDS-PAGE analysis of the purified product, CD19 Fab-FD . (D) The morphology of the nanoparticle before and after encapsulating DOX by negative-stain electron microscopy (left: CD19 Fab-F, before the encapsulation; right: CD19 Fab-FD, after the encapsulation).
Figure 8: the test of antibody-conjugated ferritin nanoparticles being thawed under different conditions after storage.
Figure 9 shows the stability tested by SDS-PAGE with Coomassie brilliant blue staining of antibody-conjugated, DOX-encapsulated ferritin nanoparticles. Samples were tested for stability by SDS-PAGE wiht Coomassie Brilliant Blue staining after storage in PBS at 4°C for 1, 2, 3, 5, and 7 weeks.
Figure 10 shows the stability of the DOX-encapsulated ferritin nanoparticles which are not conjugated with antibody. Samples were tested by FPLC after storage in PBS at 4°C for 1, 2, 3, 5, and 7 weeks.
Figure 11 shows the stability of the DOX-encapsulated ferritin nanoparticles after being conjugated with antibodies. Samples were tested by FPLC after storage in PBS or Tris buffer (10 mM Tris pH 8, 500 mM NaCl) at 4°C for 1, 2, 3, 5, and 7 weeks.
Figure 12 shows as an illustration the CD20 IgG antibody-conjugated, DOX-encapsulated ferritin nanoparticles (referred to as CD20 IgG-FD). (A) Analysis and purification by HPLC. (B) SDS-PAGE analysis of the purified product, CD20 IgG-FD.
Figure 13 shows as an illustration the CD22 IgG antibody-conjugated, DOX-encapsulated ferritin nanoparticles (referred to as CD22 IgG-FD). (A) Analysis and purification by HPLC. (B) SDS-PAGE analysis of the product, CD22 IgG-FD.
Figure 14 shows the specific binding to tumor cell lines of antibody-conjugated, DOX-encapsulated ferritin nanoparticles. The binding of various antibodies and nanoparticle-antibody conjugates with the Raji cell line (B cells) or the K562 cell line (myelogenous leukemia cells). The concentrations of the various antibodies or nanoparticle-antibody conjugates were 10, 2 and 0.4 µg/mL. (A) Flow cytometry detection of the antibody binding. (B) Histogram of MFI for each group.
Figure 15 shows that the antibody-conjugated, DOX-encapsulated ferritin nanoparticles were endocytosed by tumor cells.
Figure 16 shows the specific killing of tumor cells by the antibody-conjugated, DOX-encapsulated ferritin nanoparticles. CD19 IgG, CD19 IgG-F, CD19 IgG-FD, CD19 Fab, CD19 Fab-F, CD19 Fab-FD, CD20 IgG, CD20 IgG-F, CD20 IgG-FD, CD22 IgG, CD20 IgG-F, CD22 IgG-FD at different concentrations were tested for cell killing by CCK-8 assay.
Figure 17 shows the results of the treatment of B-cell tumor in mice. (A) Mortality rate of mice after treatment for each group; (B) Changes in body weight of mice after treatment for each group; (C) Fluorescence intensity of tumor cells in vivo shown by live imaging.

### DETAILED DESCRIPTION

The nano-delivery system described herein comprises a ferritin nanoparticle, a targeting protein, and an active molecule, for example, a therapeutic agent. The targeting protein molecule is conjugated to the ferritin protein subunit through the linker set forth in SEQ ID NO. 19, and thereby is presented on the surface of the ferritin nanoparticle to bind to a target, such as a molecule on the surface of a cell. The active molecule, for example, a therapeutic agent, is encapsulated within the ferritin nanoparticle. After the targeting protein specifically binds to the target, the nanoparticle is endocytosed into the target cell where the active molecule is released and exerts the desired active effects.

### Ferritin

Ferritin may form spherical nanoparticles with a diameter of about 12-20 nm, and is the main protein for the storage and transport of iron in organisms. Each ferritin nanoparticle is composed of 24 copies of ferritin. Ferritin can efficiently encapsulate small molecule anti-tumor drugs, thereby improving their aqueous solubility and stability in tissues (Z.G. Chen, Small-molecule delivery by nanoparticles for anticancer therapy. Trends Mol Med 16, 594-602 (2010); M. Khoshnejad, H. Parhiz, V. V. Shuvaev, I. J. Dmochowski, V. R. Muzykantov, Ferritin-based drug delivery systems: Hybrid nanocarriers for vascular immunotargeting. J Control Release 282, 13-24 (2018); Z. Wang et al., Functional ferritin nanoparticles for biomedical applications. Front Chem Sci Eng 11, 633-646 (2017)).

In the present disclosure, ferritin may be produced from ferritin-encoding genes derived from different sources including eukaryotes and prokaryotes and in the form of wild type or various modified types, which are suitable for production, capable of encapsulating active molecules (for example, therapeutic agents, such as small molecule drugs), capable of conjugating to targeting proteins by Sortase A, and suitable for administration to desired subjects, for example, cancer patients.

In some embodiments, the ferritin nanoparticle is conjugated with 24 copies of identical or different targeting proteins.

In some embodiments, modified ferritin derived from *Helicobacter pylori* having an amino acid sequence set forth in SEQ ID NO. 11 is involved, in which the N-glycosylation site at position N21 is mutated to glutamine (N21Q). Said ferritin may be used to form a spherical particle composed of 24 ferritin copies.

For the conjugation of ferritin to a targeting protein molecule by Sortase A, the amino acid sequence of GGGG (SEQ ID NO. 20) may be linked to the N-terminus of ferritin, or the encoding sequence of GGGG (SEQ ID NO. 20) may be linked to the 5' end of the encoding sequence of ferritin; and the amino acid sequence of LPXTGG (SEQ ID NO. 21) may be linked to the C-terminus of the targeting protein molecule, or the encoding sequence of LPXTGG (SEQ ID NO. 21) may be linked to the 3' end of the encoding sequence of the targeting protein molecule. After the enzymatic reaction by Sortase A, the targeting protein molecule and ferritin are conjugated together via the linker set forth in SEQ ID NO. 19, and thereby the targeting protein molecule is presented on the surface of the ferritin nanoparticle.

In some embodiments, ferritin is produced by cell lines, for example, mammalian cell lines, or by synthesis. The targeting protein is conjugated onto the surface of the ferritin nanoparticle by Sortase A and may specifically bind with a target molecule.

In some embodiments, the ferritin nanoparticle is subject to high temperature treatment, e.g., at a temperature above 50°C, to induce structural loosening, wherein the triangular pores in the nanoparticle converted into quadrilateral pores, thereby enabling the permeation of a drug to be encapsulated within the nanoparticle; subsequently, the nanoparticle is transferred to room temperature, and the quadrilateral pores revert to triangular, thereby retaining the encapsulated drug within the nanoparticle. Notably, the ferritin nanoparticle can maintain its spherical morphology even at 80°C, allowing the drug to be encapsulated to permeate into the spherical structure.

In some embodiments, the ferritin nanoparticle is subject to a strongly acidic condition e.g., below pH 3, or a strongly alkaline condition, e.g., above pH 10, to dissociate into ferritin monomers. A drug to be encapsulated is then added, and the ferritin monomers reassemble into a spherical structure under a neutral condition, e.g., around pH 7, with the drug encapsulated within the spherical structure concurrently.

In the present disclosure, ferritin may be stored at a temperature ranging from -20°C to 30°C, and subjected to repeated freeze-thaw without affecting its drug delivery function.

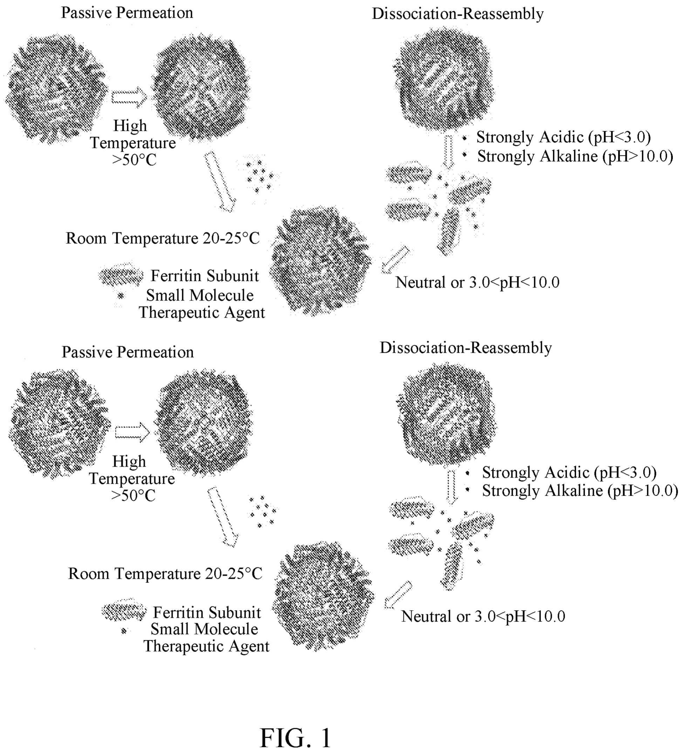

As illustrated in Figure 1, the encapsulation of a small-molecule drug by ferritin is primarily achieved through two mechanisms. One is passive permeation: under an ambient condition, e.g., 20°C to 25°C, the ferritin nanoparticle exhibits 3-fold channels (morphologically appearing as triangular pores) on its surface. When subjected to an elevated temperature, e.g. 50°C, these 3-fold channels tranform into larger 4-fold channels (morphologically appearing as quadrilateral pores), with an average diameter of 0.9 nm. Optionally thermal fluctuations may further expand the diameter to over 1 nm, allowing the transfer and encapsulation of an active molecule, e.g. a therapeutic agent. The other is dissociation-reassembly: under a strongly acidic condition, e.g., below pH 3, or a strongly alkaline condition, e.g., above pH 10, the ferritin nanoparticle disclosed herein dissociates into monomers. Upon adjustment to a neutral condition around pH 7 or a pH range of 3.0 to 10.0, those monomers reassemble into the ferritin nanoparticle, thereby enabling drug encapsulation. See, "A critical review of ferritin as a drug nanocarrier: Structure, properties, comparative advantages and challenges", Shuang Yin, Particuology 64 (2022) 65-84.

Under neutral conditions, the spherical cavity of the ferritin nanoparticle bears negative charge, enabling positively charged metal ions to readily pass through the pores of the nanoparticle and accumulate in the cavity.

In the present disclosure, the pores may be enlarged when the ferritin nanoparticle is subjected to high-temperature treatment. Furthermore, by adjusting buffer pH, the electric charge load of the cavity of the ferritin nanoparticle may be changed, and the electric charge of the drug to be encapsulated may be changed. Those treatments enable the drug to pass through the pores and enter into the cavity via electrostatic driving force. A pH value close to the pKa may facilitate the deposition of the drug in the cavity of the ferritin nanoparticle.

In the present disclosure, the encapsulation of the drug by the ferritin nanoparticle may be achieved by adjusting temperature or pH.

### Sortase A

Sortase A may, in the presence of Ca²⁺ and at an optimal pH (7.0-9.0), mediate an acylation reaction to conjugate two peptide or protein molecules together. That is, N terminal-GGGG-protein 1 and C-terminal LPXTGG-protein 2 produce protein 2-LPXTGGGG-protein 1. Sortase A is widely used in the field of genetic engineering for the precise insertion or conjugation of non-natural molecules, such as fluorescein or amides, into proteins to alter their physical or chemical properties.

For the linker set forth in LPXTGGGG (SEQ ID NO. 19) formed by Sortase A, X can be any amino acid, for example M (Met), A (Ala), V (Val), L (Leu), I (Ile), C (Cys), S (Ser), T (Thr), N (Asn), Q (Gln), D (Asp), E (Glu), H (His), K (Lys), R (Arg), G (Gly), P (Pro), W (Trp), Y (Tyr) or F (Phe). In other words, the conjugation between two peptide or protein molecules may be achieved by Sortase A, when the amino acid sequence of GGGG (SEQ ID NO. 20) is linked to the N-terminus of one molecule and the amino acid sequence of LPXTGG (SEQ ID NO. 21) is linked to the C-terminus of the other.

In some embodiments, to achieve the conjugation between ferritin and the targeting protein molecule disclosed herein, the amino acid sequence of GGGG (SEQ ID NO. 20) is linked to the N-terminus of ferritin, and the amino acid sequence of LPXTGG (SEQ ID NO. 21) is linked to the C-terminus of the targeting protein molecule, wherein X may be any amino acid, e.g. E. In some embodiments, when the targeting molecule is an antibody, e.g. IgG antibody, the amino acid sequence of LPXTGG (SEQ ID NO. 21) may be linked to the C-terminus of the heavy chain, thereby enabling conjugation to ferritin.

### Active molecule

In the present disclosure, any suitable active molecule. e.g., a therapeutic agent, such as a small molecule drug, may be encapsulated in the ferritin nanoparticle to form a nano-delivery system for targeted delivery.

In some embodiments, more than one active molecule, e.g., therapeutic agent, is used simultaneously. In some embodiments, for example, suitable active molecules, e.g., therapeutic agents, include, but are not limited to, anthracycline drugs, such as doxorubicin, daunorubicin, epirubicin, and idarubicin; antimetabolite chemotherapeutic agents, such as 5-fluorouracil, methotrexate, capecitabine, azacitidine, and acivicin; B-cell lymphoma-2 (Bcl-2) inhibitors, such as venetoclax, ABT-737, or navitoclax; tyrosine kinase inhibitors, such as gefitinib, erlotinib, icotinib, afatinib, dacomitinib, lapatinib, and almonertinib; platinum-based chemotherapeutic agents, such as cisplatin; Borofalan-10B.

In some embodiments, doxorubicin is encapsulated in the ferritin nanoparticle for targeted delivery into target cells, e.g., tumor cells. In some embodiments, cisplatin is encapsulated in the ferritin nanoparticle for targeted delivery into target cells, e.g., tumor cells.

### Targeting protein

A targeting protein refers to a protein molecule that specifically binds a target molecule or a cell-associated protein, e.g., a cell surface molecule. It is typically used as a drug for treating disease.

As used herein, the targeting protein is linked at the C-terminus to the amino acid sequence set forth in SEQ ID NO: 21 (LPXTGG), thereby enabling it to be conjugated by Sortase A to the amino acid sequence set forth in SEQ ID NO: 20 (GGGG) at the N-terminus of ferritin, forming the linker set forth in SEQ ID NO: 19 (LPXTGGGG). This process allows the targeting protein to be displayed on the surface of the ferritin nanoparticle. The targeting protein may be produced by cell lines, e.g., mammalian cells.

In some embodiments, the targeting protein may be an antibody molecule or an antigen-binding fragment thereof. To achieve the conjugation to ferritin, the amino acid sequence of LPXTGG (SEQ ID NO. 21) is linked to the C-terminus of the heavy chain of the antibody molecule or the antigen-binding fragment thereof, or the encoding sequence of the amino acid sequence of LPXTGG (SEQ ID NO. 21) is linked to the 3' end of the encoding sequence of the heavy chain of the antibody molecule or the antigen-binding fragment thereof. Any suitable antibody molecule or its antigen-binding fragment, e.g., Fab, may be used, such as a monoclonal antibody targeting CD19, CD20, or CD22 or an antigen-binding fragment thereof.

In some embodiments, 24 identical or different targeting protein molecules may be conjugated to a single ferritin nanoparticle.

### Use

The nano-delivery system described herein may target different cells, molecules, or environments by conjugating different targeting protein molecules, and release the encapsulated active molecules, e.g., therapeutic agents, thereby exerting desired effects, for example, treating desired disease.

The nano-delivery system described herein may be used for various purposes. For example, it may be used to treat non-Hodgkin's lymphoma or lymphocytic leukemia by conjugating to CD19, CD20, or CD22 antibodies, or treat non-small cell lung cancer, colorectal cancer, head and neck cancer, breast cancer or the like by conjugating with EGFR or HER2 antibodies.

The nano-delivery system of the present disclosure enables the provision of a safe, stable, low-toxic, strongly targeting, multi-specific therapy.

### Examples

The technical solutions of the disclosure will be explained below in conjunction with examples. Those skilled in the art will understand that the following examples are only used for illustrative purposes only and should not be understood as limiting the scope of the invention.

For specific techniques or conditions not indicated in the examples, they are carried out according to the techniques or conditions described in literatures in the field or according to the product or instrument instructions. All reagents or instruments indicating manufacturers are available commercially.

The various antibodies or nanoparticle-antibodies involved in the examples are as follows: CD19 IgG, CD19 IgG-conjugated ferritin nanoparticles (CD19 IgG-F), CD19 IgG-conjugated, DOX-encapsulated ferritin nanoparticles (CD19 IgG-FD), CD19 Fab, CD19 Fab-conjugated ferritin nanoparticles (CD19 Fab-F), CD19 IgG-conjugated, DOX-encapsulated ferritin nanoparticles (CD19 Fab-FD), CD20 IgG, CD20 IgG-conjugated ferritin nanoparticles (CD20 IgG-F), CD20 IgG conjugated, DOX-encapsulated ferritin nanoparticles (CD20 IgG-FD), CD22 IgG, CD22 IgG-conjugated ferritin nanoparticles (CD20 IgG-F), CD22 IgG-conjugated, DOX-encapsulated ferritin nanoparticles (CD22 IgG-FD).

### Example 1. Production and assembly of ferritin

The ferritin used in the examples is derived from *Helicobacter pylori* with mutation. The mutated encoding sequence and amino acid sequence are set forth in SEQ ID NO. 2 and SEQ ID NO. 11, respectively, as shown below, with the N21Q mutation in the sequences is marked in bold and underlined.

Given the mammalian cell expression system is closely homologous to humans, the expressed products are most representative of the genuine *in vivo* biological activity. The inventor found that mutating the asparagine residue at position 21 to glutamine (N21Q) effectively abolishes the N-glycosylation modification at this site. This mutation not only allows the ferritin expressed in mammalian cells to maintain a conformation more closely resembling its native status, but also achieves a high yield of more than 80mg/L cells.

### 1) Expression and purification of ferritin

The nucleotide sequence of SEQ ID No. 2 was cloned into the expression vector pcDNA3.1, and the recombinant plasmid was obtained and designated as pcDNA3.1-Ferritin_Sec. 293F cells were transfected with the plasmid pcDNA3.1-Ferritin_Sec using polyethyleneimine (PEI). Four days after transfection, the cells were collected via centrifugation. The lysis solution (150 mM NaCl, 25 mM Tris pH=8, 0.0001% IGEPAL^{®} CA-630, protease inhibitors (Beyotime Biotechnology, catalog number P1006)) was added. After three cycles of freeze-thaw, the supernatant was collected by centrifugation, followed by purification using the Histrap Column (Cytiva, catalog number 29-0510-21) and HiLoad 16/600 Superdex 200pg molecular sieve chromatography(Cytiva, catalog number 28-9893-35).

### 2) Assembly of ferritin nanoparticles

A single ferritin nanoparticle is composed of 24 ferritin subunits. To present the targeting protein antibodies on the surface of the ferritin nanoparticles, the linker of GGGG were added to the N-terminus of each subunit (Figure 3A). The ferritin nanoparticles after cell expression and purification were separated by HPLC, showing a single peak (Figure 3B). The ferritin nanoparticles after cell expression and purification showed uniformly spherical structure by TEM (Figure 3C). Collectively, those data indicate that ferritin can efficiently assemble into nanoparticles.

We tested the assembly ability of ferritin into nanoparticles under different pH and temperatures.

In a strong acidic solution with pH less than 3.0, the ferritin nanoparticles dissociated into ferritin monomers, and then by replacing the solution with a neutral PBS solution, ferritin reassembled into nanoparticles consistent with the previous form (Figure 4A). A strong alkaline solution with pH more than 10.0 also caused the ferritin nanoparticles to dissociate into ferritin monomers, and by replacing the solution with neutral PBS solution, the ferritin reassembled into nanoparticles consistent with the previous form (Figure 4B).

We placed the ferritin nanoparticles at high temperature greater than 50°C (e.g., 60°C), and found that upon returning to room temperature, the size of the nanoparticles remained consistent with their previous size (Figure 4C).

These results indicate that the ferritin nanoparticles in the present disclosure have good potential for delivering small molecule drugs.

### 3) Comparison between human ferritin and Helicobacter pylori ferritin

The comparison between *Helicobacter pylori* ferritin and human ferritin for encapsulating the small molecule drug doxorubicin (DOX) was performed. The amount of DOX encapsulated in the ferritin nanoparticles was determined by the the absorbance at OD480 with NanoDrop, and the concentration of ferritin was determined by the absorbance at OD280 with NanoDrop (Zhang J., Cheng D., He J. et al. Cargo loading within ferritin nanocages in preparation for tumor-targeted delivery. Nat Protoc 16, 4878-4896 (2021)).

Specifically, 1 mg ferritin nanoparticles was thawed at room temperature, and 790 µl of 50 mM Tris-HCl pH9.0 was added and mixed well, followed by the addition of 0.3 mg Dox. The total volume was 1 mL. The mixture was transfered to pate wells, covered with tin foil to protect from light, and incubated at 60°C for 1 hour. The mixture was then transferred to room temperature (20°C-25°C), 1 ml of dH₂O at room-temperature was added, and the mixture was homogenized before being transferred to a 3K 15-ml ultrafiltration tube (Pall Corporation, catalog number MCP003C46). During ultrafiltration, the mixture was buffer-exchanged and concentrated in 0.5 ml of 10 mM Tris, 500 mM NaCl, pH8.0, followed by filtering through a 0.2 µm filter membrane for subsequent detection or storage in aliquots. The results are shown in Table 1 below.

**Table 1**

| | DOX/ferritin (OD480) | Ferritin Recovery Rate(%) |
|---|---|---|
| *Helicobacter pylori* ferritin + DOX | 61 ± 17 | 86 ± 4 |
| Human ferritin + DOX | 53 ± 12 | 84 ± 6 |

According to the results in Table 1, the *Helicobacter pylori* ferritin is higher than or comparable to human ferritin in terms of the number of the encapsulated DOX molecules and the ferritin recovery rate, and it is a good carrier for delivering small molecule drugs.

### Example 2. Preparation of nano-delivery systems conjugated with CD19 antibody and antigen-binding fragment fab thereof

### 1) Preparation of Sortase A

For ease of use, Sortase A was expressed and purified in our laboratory. However, it is understood that any commercially available Sortase A may be used.

The sequence set forth in SEQ ID No. 1 was cloned into the expression vector pcDNA3.1. After extraction, the recombinant plasmid was obtained and , designated as pcDNA3.1-SrtA7_Int. 293F cells were tranfected with the plasmid pcDNA3.1-SrtA7_Int using PEI. Four days post-transfection, the cells were collected via centrifugation. The lysis solution (150 mM NaCl, 25 mM Tris pH=8, 0.0001% IGEPAL^{®} CA-630, protease inhibitors (Beyotime Biotechnology, catalog number P1006)) was added. After three cycles of freeze-thaw, the supernatant was collected by centrifugation, followed by purification using the Histrap Column (Cytiva, catalog number 29-0510-21) and HiLoad 16/600 Superdex 200pg molecular sieve chromatography (Cytiva, catalog number 28-9893-35).

### 2) Preparation of recombinant antibody and its antigen-binding fragment

For ease of use, the CD19 IgG antibody and its antigen-binding fragment were expressed and purified in our laboratory. To enable the Sortase A-mediated conjugation to ferritin, the amino acid sequence of LPETGG was linked to the C-terminus of the antibody heavy chain.

The sequences of SEQ ID NOs. 3, 4 and 5 were cloned into the expression vector pcDNA3.1, respectively. After extraction, the recombinant plasmids were obtained and designated as pcDNA3.1-CD19IgH, pcDNA3.1-CD19FabH, and pcDNA3.1-CD19IgK.

To express the CD19 IgG antibody, 293F cells were transfected using PEI with the plasmids pcDNA3.1-CD19IgH and pcDNA3.1-CD19IgK encoding the heavy and light chains of the CD19 antibody. Four days post-transfection, the supernatant was collected by centrifugation and purified using Protein A Antibody purification column (Cytiva, catalog number 29497628). The purified product was then subjected to a second purification using HiLoad 16/600 Superdex 200pg molecular sieve chromatography (Cytiva, catalog number 28-9893-35).

Similarly, to express the antigen-binding fragment Fab of the CD19 antibody, 293F cells were transfected using PEI with the plasmids pcDNA3.1-CD19FabH and pcDNA3.1-CD19IgK encoding the CD19 antibody Fab fragment. Four days post-transfection, the supernatant was collected by centrifugation and purified using Protein L affinity resin (Yeasen Biotech, catalog number 36407ES08). The purified product was then subjected to a second purification using HiLoad 16/600 Superdex 200pg molecular sieve chromatography (Cytiva, catalog number 28-9893-35).

### 3) Preparation of small molecule drug-encapsulated ferritin nanoparticles

1 mg of ferritin nanoparticles was thawed at room temperature, 790 µl of 50 mM Tris-HCl pH9.0 was added and mixed well, followed by the addition of 0.3 mg Dox. The total volume was 1 mL. The mixture was transfered to pate wells, covered with tin foil to protect from light, and incubated at 60°C for 1 hour. 1 ml of dH2O at room-temperature was added, and the mixture was homogenized before being transferred to a 3K 15-ml ultrafiltration tube (Pall Corporation, catalog number MCP003C46). During ultrafiltration, the mixture was buffer-exchanged and concentrated in 10 mM Tris, 500 mM NaCl, pH8.0, followed by filtering through a 0.2 µm filter membrane for subsequent detection or storage in aliquots.

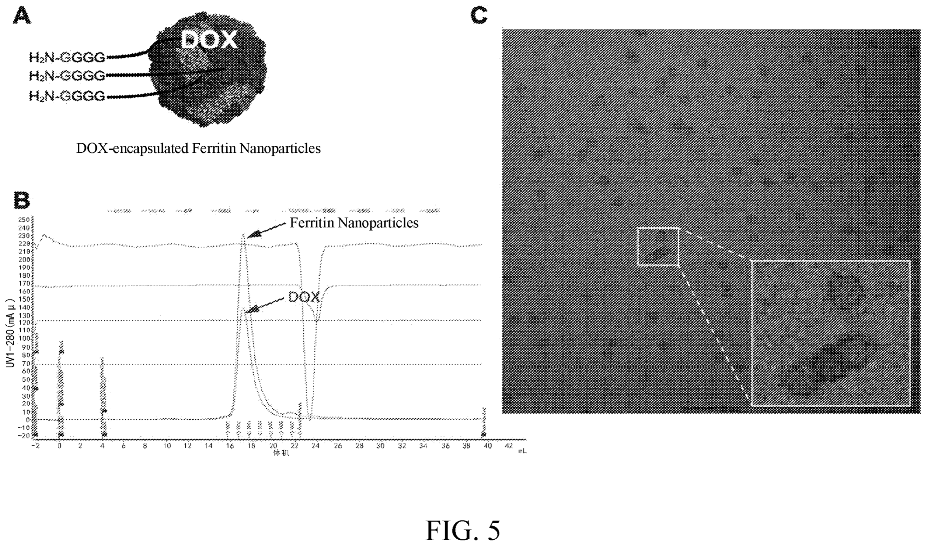

The small molecule drug DOX was successfully encapsulated within the nanoparticles via a high-temperature treatment (60°C for 1 hour) followed by reassembly at room temperature (20-25°C) (Figure 5A). HPLC analysis revealed the peak profile of the ferritin nanoparticles completely consistent with that of DOX (Figure 5B). The DOX-encapsulated ferritin nanoparticles exhibited uniformly spherical structures with a darker interior by TEM (Figure 5C). Collectively, the data demonstrate that ferritin nanoparticles can efficiently encapsulate small molecule drugs such as DOX.

### 4) Conjugation of antibodies to small molecule drug-encapsulated ferritin nanoparticles

The reaction system was prepared in Vivaspin 20 tube by adding DOX-encapsulated ferritin nanoparticles at the final concentration of 120 µM, 120 µM CD19 IgG antibody, 100 µM Sortase A, and the reaction buffer (50 mM Tris, 150 mM NaCl, 5 mM CaCl₂, pH 7.5). Following centrifugation, the reaction system was concentrated to 250 µL and transferred to a 500 µL tube, then placed on a shaker at room temperature overnight. Subsequently, purification was performed using HiLoad 16/600 Superdex 200pg molecular sieve chromatography.

The CD19 IgG antibody we prepared has the tag of LPETGG at the C-terminus of the heavy chain, enabling the conjugation to the DOX-encapsulated ferritin nanoparticles from the previous step via the catalytic action of Sortase A (Figure 6A). HPLC anaylsis revealed the peak profile of the nanoparticles completely consistent with that of DOX. Additionally, a peak of unconjugated IgGs and a peak of Sortase A were observed (Figure 6B). SDS-PAGE analysis with Coomassie Brilliant blue staining showed four bands which were identified as ferritin-CD19 IgG heavy chain, CD19 IgG heavy chain, CD19 IgG light chain, and ferritin, indicating high purity (Figure 6C). TEM analysis revealed spherical particles with serrated edges (Figure 6D), suggesting the surface display of antibodies on the nanoparticles .

### 5) Conjugation of antigen-binding fragment Fab to small molecule drug-encapsulated ferritin nanoparticles

Similarly, the CD19 antigen-binding fragment Fab was conjugated onto the surface of the ferritin nanoparticles via the catalytic action of Sortase A (Figure 7A). HPLC analysis revealed the peak profile of the nanoparticles completely consistent with that of DOX. Additionally, a peak of unconjugated CD19 Fab and Sortase A was also observed (Figure 7B). SDS-PAGE analysis with Coomassie Brilliant blue staining showed four bands which were identified as ferritin-conjugated Fab heavy chain, unconjugated Fab heavy chain, Fab light chain, and unconjugated ferritin (Figure 7C). TEM analysis revealed spherical nanoparticles with serrated edges (Figure 7D), indicating the surface display of the fragments on the nanoparticles.

Collectively, the data confirm the successful conjugation of the fragment to the nanoparticles and the efficient encapsulation of DOX within the nanoparticles. Such nanoparticle exhibits the specificity of the antibody fragment, enabling the targeted delivery of small-molecule anti-tumor drugs to tumor cells via specific targets on their surface.

### 6) Temperature stability evaluation of antibody-conjugated, small molecule drug-encapsulated ferritin nanoparticles.

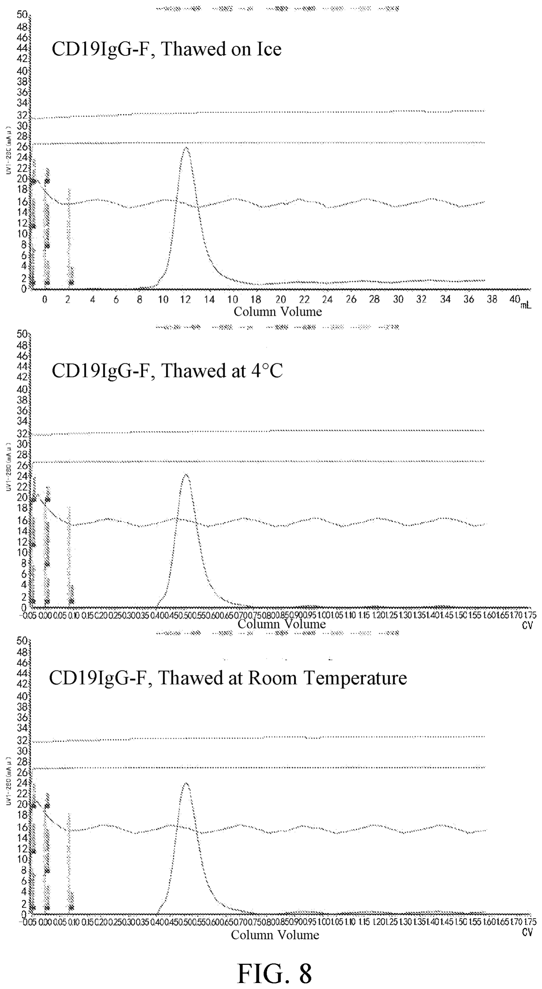

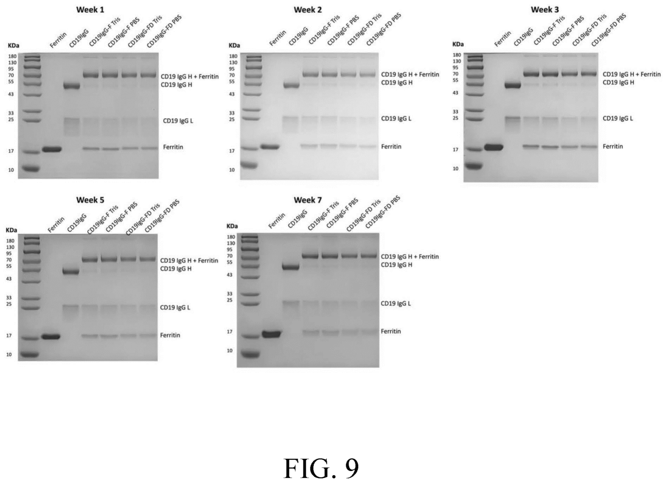

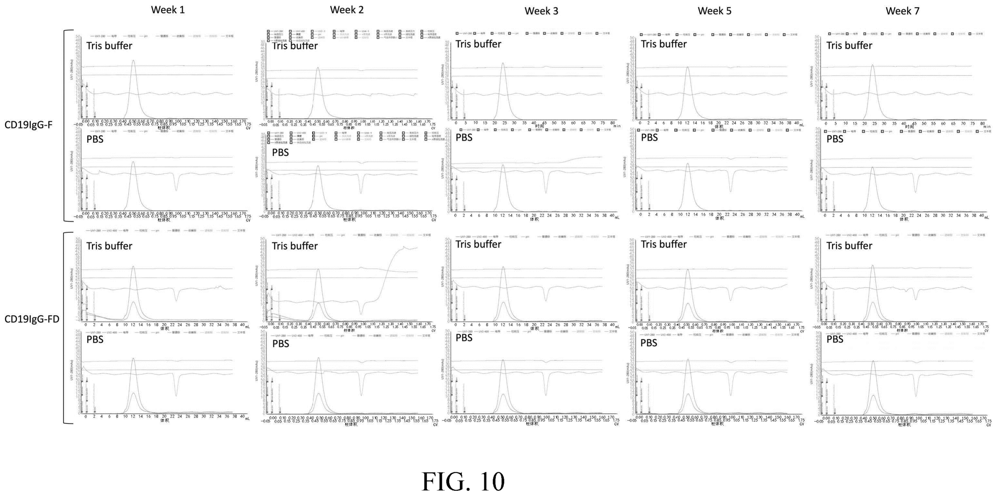

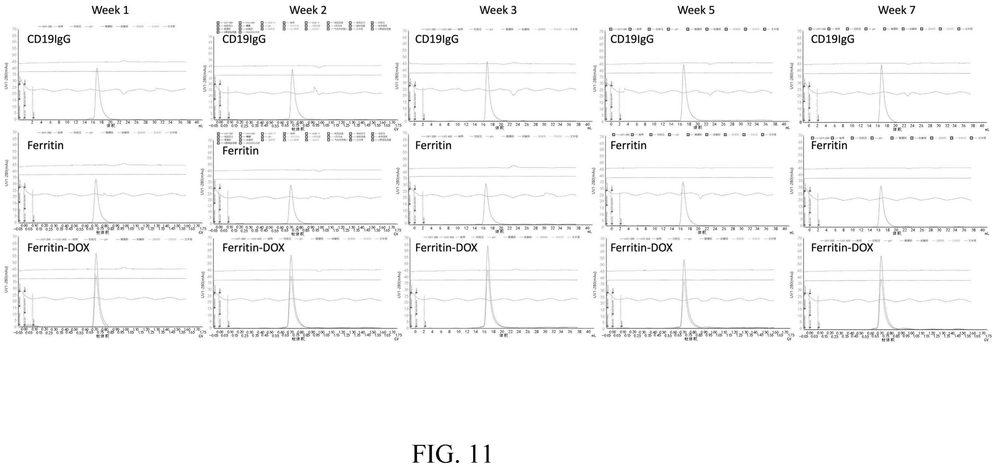

To evaluate the freeze-thaw stability of the antibody-conjugated nanoparticles, CD19 IgG-ferritin nanoparticles (i.e. CD19 IgG-conjugated ferritin nanoparticles) stored at -80°C were thawed on ice, at 4°C, and at room temperature, respectively. HPLC analysis revealed no degradation of the CD19 IgG-conjugated ferritin nanoparticles after thawing (Figure 8). Subsequently, the nanoparticles were stored in PBS at 4°C for 1, 2, 3, 5, and 7 weeks, respectively. Stability was evaluated by SDS-PAGE with Coomassie Brilliant blue staining (Figure 9) and HPLC (Figure 10). The results indicated that the antibody-conjugated ferritin nanoparticles remained stable for at least 7 weeks when stored at 4°C. Consistent results were obtained when PBS was replaced with Tris buffer, demonstrating that DOX-encapsulated, CD19 IgG-conjugated ferritin nanoparticles can maintain stability for at least 7 weeks at 4°C in either PBS or Tris buffer (Figure 11).

### Example 3: Preparation of nano-delivery systems conjugated to CD20 IgG antibody and CD22 IgG antibody

### 1) Preparation of recombinant antibodies and antigen-binding fragments thereof

For ease of use, the CD20 IgG and CD22 IgG antibodies, both with the sequence of LPETGG linked to the C-terminus, were expressed and purified in our laboratory.

The sequences of SEQ ID Nos. 6, 7, 8 and 9 were cloned into the expression vector pcDNA3.1. After plasmid extraction, the recombinant plasmids were obtained and designated as pcDNA3.1-CD20IgH, pcDNA3.1-CD20IgK, pcDNA3.1-CD22IgH and pcDNA3.1-CD22IgK.

To express the CD20 IgG and CD22 IgG antibodies, 293F cells were transfected using PEI with the plasmids pcDNA3.1-CD20IgH and pcDNA3.1-CD20IgK encoding the heavy and light chains of the CD20 antibody, as well as with the plasmids pcDNA3.1-CD22IgH and pcDNA3.1-CD22IgK encoding the heavy and light chains of the CD22 antibody. Four days post-transfection, the supernatant was collected by centrifugation and purified using Protein A Antibody purification column (Cytiva, catalog number 29497628). The purified product was then subjected to a second purification using HiLoad 16/600 Superdex 200pg molecular sieve chromatography (Cytiva, catalog number 28-9893-35).

### 2) Preparation of small molecule drug-encapsulated ferritin nanoparticles

1 mg of ferritin nanoparticles was thawed at room temperature, 790 µl of 50 mM Tris-HCl pH9.0 was added and mixed well, followed by the addition of 0.3 mg Dox. The total volume was 1 mL. The mixture was transfered to pate wells, covered with tin foil to protect from light, and incubated at 60°C for 1 hour. 1 ml of room-temperature dH2O was added, and the mixture was homogenized before being transferred to a 3K 15-ml ultrafiltration tube (Pall Corporation, catalog number MCP003C46). During ultrafiltration, the mixture was buffer-exchanged and concentrated in 10 mM Tris, 500 mM NaCl, pH8.0, followed by filtering through a 0.2 µm filter membrane for subsequent detection or storage in aliquots.

### 3) Conjugation of antibody to small molecule drug-encapsulated ferritin nanoparticles

The reaction system was prepared in Vivaspin 20 tube by adding ferritin nanoparticles at final concentration of 120 µM, 120 µM CD20 IgG or CD22 IgG antibody, 100 µM Sortase A and the reaction buffer (50 mM Tris, 150 mM NaCl, 5 mM CaCl2, pH 7.5). Following centrifugation, the reaction system was concentrated to 250 µL and transferred to a 500 µL tube, then placed on a shaker at room temperature overnight. Subsequently, purification was performed using HiLoad 16/600 Superdex 200pg molecular sieve chromatography.

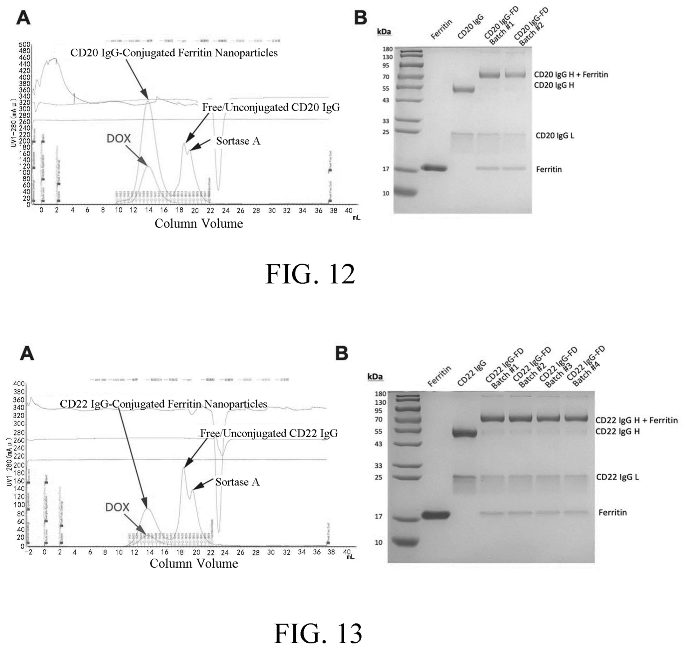

The CD20 IgG and CD22 IgG antibodies we prepared have the tag of LPETGG at the C-terminus of the heavy chain, enabling the conjugation to the DOX-encapsulated ferritin nanoparticles from the previous step via the catalytic action of Sortase A. HPLC anaylsis revealed the peak profile of the nanoparticle completely consistent with that of DOX. Additionally, a peak of unconjugated IgGs and a peak of Sortase A were observed (Figure 12A, Figure 13A). SDS-PAGE analysis with Coomassie Brilliant blue staining showed four bands which were identified as IgG heavy chain-conjugated ferritin, IgG heavy chain, IgG light chain, and ferritin, indicating high purity (Figure 12B, Figure 13B).

### Example 4: Surface binding and endocytosis assays of B-cell tumor cell lines

### 1) Cell surface binding assay

The following antibodies and antibody-conjugated nanoparticles were prepared as described above: CD19 IgG, CD19 IgG-conjugated ferritin nanoparticles (CD19 IgG-F), CD19 IgG-conjugated, DOX-encapsulated ferritin nanoparticles (CD19 IgG-FD), CD19 Fab, CD19 Fab-conjugated ferritin nanoparticles (CD19 Fab-F), CD19 IgG-conjugated, DOX-encapsulated ferritin nanoparticles (CD19 Fab-FD), CD20 IgG, CD20 IgG-conjugated ferritin nanoparticles (CD20 IgG-F), CD20 IgG-conjugated, DOX-encapsulated ferritin nanoparticles (CD20 IgG-FD), CD22 IgG, CD22 IgG-conjugated ferritin nanoparticles (CD20 IgG-F), and CD22 IgG-conjugated, DOX-encapsulated ferritin nanoparticles (CD22 IgG-FD).

Antibodies and antibody-conjugated nanoparticle samples were diluted to 10, 2 or 0.4 µg/mL, and incubated with 1x10⁵ Raji cells (B cell line) or K562 cells (myelogenous leukemia cells) on ice for 30 minutes, followed by washing once with PBS. Then, Alexa Fluor 488 anti-human IgG (H+L) (ThermoFisher, catalog number A-11013) diluted 1:2000 was added to the mixture. The mixture were incubated on ice for 30 minutes followed by washing twice with PBS and fixing with 1% formaldehyde. The binding of the antibodies and antibody-encapsulated nanoparticle to Raji cell line or K562 cell line was evaluated using flow cytometry.

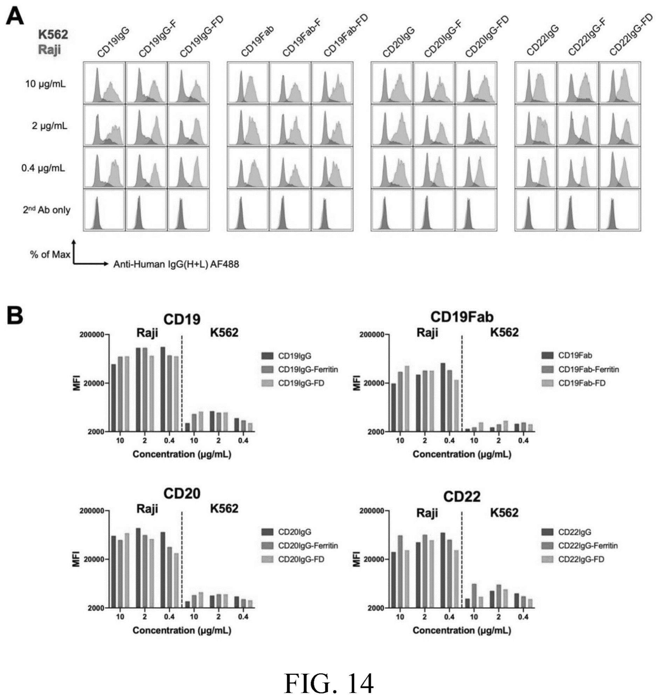

As shown in Figure 14A and 14B, the antibodies can efficiently bind to Raji cells at concentrations of 10, 2, and 0.4 µg/mL, whereas no binding to K562 cells was observed. This is attributed to the surface expression of antigens CD19, CD20, and CD22 on Raji cells, while the control K562 cells lack the expression. Therefore, the antibody conjugated, DOX-encapsulated nanoparticles can specifically bind to and target Raji cells.

### 2) Cell endocytosis assay

Antibody encapsulated nanoparticle samples (CD19 IgG-F, CD19 IgG-FD, CD19 Fab-F and CD19 Fab-FD) were diluted to 10µg/mL. Each diluted sample was mixed with 1x10⁵ Raji cells, and incubated for 30 minutes or 120 minutes. Following incubation, the mixtures were fixed with 1% formaldehyde plus 1% acetone, then washed three times with PBS. Subsequently, Alexa Fluor 488 anti-human IgG (H+L) (ThermoFisher, catalog number A-11013), 1:1000 diluted, was added to each sample. The mixtures were incubated for 30 minutes, washed twice with PBS and visualized by fluorescence confocal microscopy.

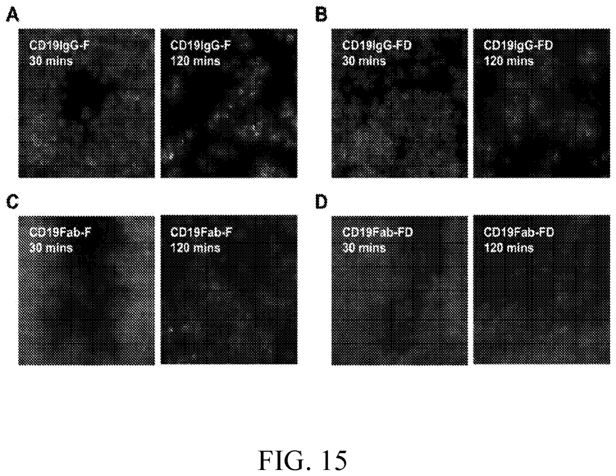

The fluorescence confocal microscopy revealed that the nanoparticles CD19 IgG-F, CD19 IgG-FD, CD19 Fab-F, CD19 Fab-FD predominantly bound to the cell surface after incubation with Raji cells for 30 min, and the majority of the nanoparticles had been endocytosed into the cytoplasm of the cells after incubation for 120 min (Figure 15).

### Example 5: Specific killing of B-cell tumor cell lines

Following evaluation of specific binding and endocytosis, the antibody-conjugated nanoparticles were incubated with Raji cells and K562 cells at 37°C at different concentrations, and further evaluated as anti-tumor drugs for their specific killing of B-cell tumors.

Specifically, Raji and K562 cells were seeded in 96-well plates at a density of 3x10⁴ cells/ml in 100 µl medium. Subsequently, ferritin nanoparticles, DOX-encapsulated ferritin nanoparticles, antibodies, antibody-conjugated ferritin nanoparticles, or antibody conjugated, DOX-encapsulated ferritin nanoparticles (FD) were added. The maximum total protein concentration is 50 µg/ml, in fivefold serial dilutions. After 72 hours of incubation, CCK-8 assay (Novizan, catalog number A311) was performed, and the absorbance at OD450 was measured 4 hours after the addition of the CCK-8 reagent.

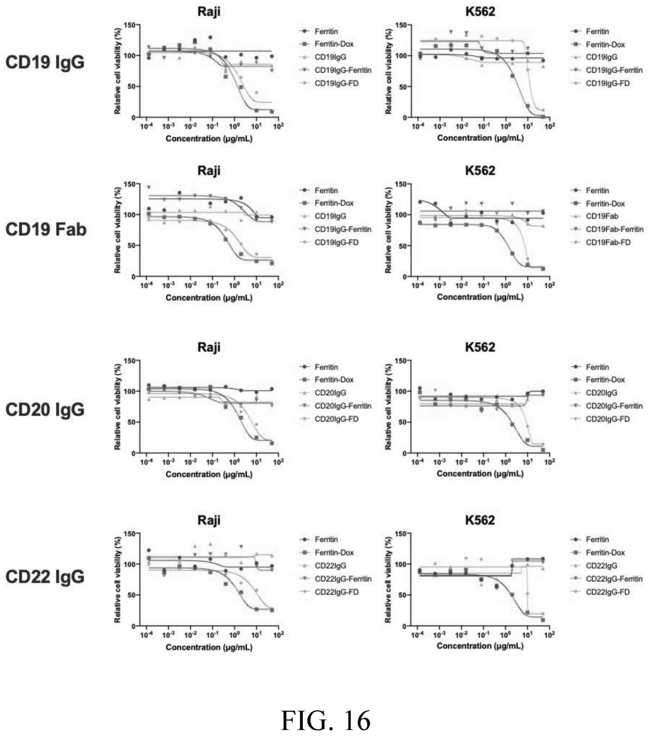

As shown in Figure 16, samples lacking DOX exhibited no cytotoxicity against either Raji cells or K562 cells. DOX-encapulated Ferritin nanoparticles had comparable cytotoxic effects on both Raji cells and K562 cells. CD19 IgG-FD, CD19 Fab-FD, CD20 IgG-FD, and CD22 IgG-FD showed stronger cytotoxicity against Raji cells, while only weak cytotoxicity was observed against the control K562 cells. Cytotoxic effects on K562 cells were only observed at the highest concentration, which is attributed to the absence of the corresponding antigens on the surface of K562 cell. These results demonstrate that the antibody-encapsulated nanoparticles of the present invention have specific cytotoxicity against B-cell tumors.

### Example 6: In vivo treatment of B-cell tumor

Female 6-week-old B-NDG mice were inoculated with B-luc-GFP Raji cells via tail vein at a dose of 1x10⁵ cells/mouse. When the average luciferase signal detected by live imaging reached 1x10⁶ P/S, the mice were subjected to different treatments (as shown in Table 2 below). After the administration of the test agents, body weight was measured daily, and live imaging bioluminescence intensity was measured twice a week. In accordance with animal welfare principles, mice were euthanized when body weight decreased by more than 20%. The results are shown in Table 2 below.

**Table 2**

| **Group** | **Number of Animals** | **Treatment** | **Drug Dose (calculated based on the total protein concentration)** | **Drug Dose (based on DOX)** | **administration Volume** | **Administration Route** | **Administration Time (after tumor inoculation)** |
|---|---|---|---|---|---|---|---|
| 1 | 7 | PBS | - | - | 200 µ L/mouse | i.v. | D3, D5, D7 |
| 2 | 5 | Ferritin | 100 µ g/mouse | - | 1200 µ L/mouse | i.v. | D3, D5, D7 |
| 3 | 6 | Ferritin-Dox | 100 µ g/mouse | 8.8 µ g/mouse | 200 µ L/mouse | i.v. | D3, D5, D7 |
| 4 | 6 | CD19 IgG-F | 100 µ g/mouse | - | 1200 µ L/mouse | i.v. | D3, D5, D7 |
| 5 | 6 | CD19 IgG-FD | 100 µ g/mouse | 3.7 µ g/mouse | 200 µ L/mouse | i.v. | D3, D5, D7 |
| 6 | 6 | CD19 IgG-FD | 50 µ g/mouse | 1.85 µ g/mouse | 200 µ L/mouse | i.v. | D3, D5, D7 |
| 7 | 6 | CD20 IgG-FD | 50 µ g/mouse | 1.85 µ g/mouse | 200 µ L/mouse | i.v. | D3, D5, D7 |

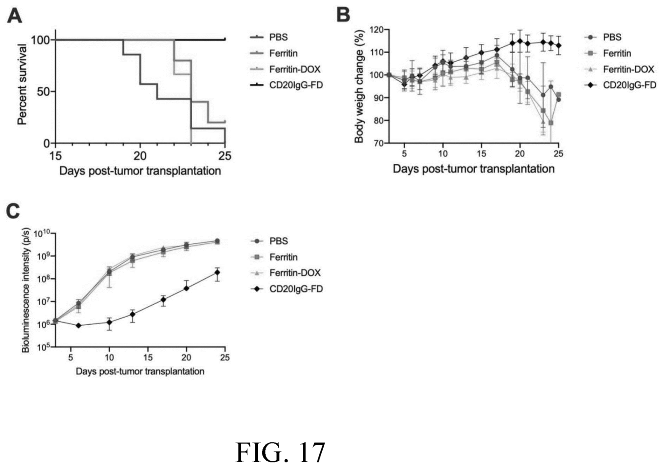

The results are shown in Figure 17, demonstrating that B-luc-GFP Raji cells successfully formed tumors in mice, leading to body weight loss and death. In vivo imaging revealed significant bioluminescence signals resulted from tumor cell proliferation. Mice administered with CD20 IgG-conjugated, DOX-encapsulated ferritin nanoparticles (CD20 IgG-FD) all survived (Figure 17A), showed no body weight loss (Figure 17B), and exhibited significantly lower in vivo bioluminescence intensity compared to mice in other groups (Figure 17C). These findings demonstrate that CD20 IgG-FD can effectively treat Raji B-cell tumors formed in mice.

Table 3 summarizes the results on Day 14 after administration. Compared with the control group, neither the ferritin 100 µg/mouse group nor the ferritin-DOX 100 µg/mouse group showed inhibitory effects on tumor growth. In contrast, the groups of CD19 IgG-ferritin 100 µg/mouse, CD19 IgG-FD 100 µg/mouse, CD19 IgG-FD 50 µg/mouse and CD20 IgG-FD 50 µg/mouse all exhibited significant inhibition of tumor growth (TGI: tumor growth inhibition rate, p<0.05).

**Table 3. Effects of on the tumor growth in B-lucGFP Raji cell-implanted B-NDG mice.**

| **Test agents** | **Tumor Imaging Signal Intensity (p/sec)^{a}** | | | **p ^{b}** |
|---|---|---|---|---|
| | **Before Administration** | **Day 14 After Administration** | **TGI (%)** | |
| PBS | 1.50 × 10⁶ ± 1.05 × 10⁵ | 1.88 × 10⁹ ± 9.07 × 10⁷ | - | - |
| Ferritin (100 µ g/mouse) | 1.33 × 10⁶ ± 9.44 × 10⁴ | 1.49 × 10⁹ ± 2.49 × 10⁸ | 20.7 | 0.6030 |
| Ferrtin-Dox (100 µ g/mouse) | 1.43 × 10⁶ ± 1.25 × 10⁵ | 2.39 × 10⁹ ± 1.51 × 10⁸ | -26.8 | 0.2549 |
| CD19 IgG-Ferritin (100 µ g/mouse) | 1.43 × 10⁶ ± 1.24 × 10⁵ | 1.05 × 10⁹ ± 1.21 × 10⁸ | 44.5 | *0.0104 |
| CD19 IgG-FD (100 µ g/mouse) | 1.44 × 10⁶ ± 1.18 × 10⁵ | 7.21 × 10⁸ ± 1.28 × 10⁸ | 61.8 | ***0.0002 |
| CD19 IgG-FD (50 µ g/mouse) | 1.44 × 10⁶ ± 1.32 × 10⁵ | 1.07 × 10⁹ ± 9.59 × 10⁷ | 43.3 | *0.0137 |
| CD20 IgG-FD (50 µ g/mouse) | 1.43 × 10⁶ ± 1.21 × 10⁵ | 1.21 × 10⁷ ± 2.40 × 10⁶ | 99.4 | ****<0.000 1 |

| | | | | |
|---|---|---|---|---|
| Note: ^{a}: Mean ± standard error; ^{b}: Statistical comparison of body weight between each treatment group and the PBS control group were performed on day 14 after administration using One-way ANOVA analysis, followed by Dunnett test for intergroup comparison. *p<0.05, p<0.01,p<0.001,***p<0.0001. | | | | |

### Sequences

SEQ ID No.1: SRTA7_INT 
ATGCACCATCATCATCATCATTCTTCTGGTCTGGTGCCACGCGGTTCTGG
TATGAAAGAAACCGCTGCTGCTAAATTCGAACGCCAGCACATGGACAG
CCCAGATCTGGGTACCCAAGCTAAACCTCAAATTCCGAAAGATAAATCG
AAAGTGGCAGGCTATATTGAAATTCCAGATGCTGATATTAAAGAACCAG
TATATCCAGGACCAGCAACACGGGAACAATTAAATAGAGGTGTAAGCTT
TGCAAAGGAAAATGCCTCACTAGATGATCAAAATATTTCAATTGCAGGA
CACACTTTCATTGACCGTCCGAACTATCAATTTACAAATCTTAAAGCAG
CCAAAAAAGGTAGTATGGTGTACTTTAAAGTTGGTAATGAAACACGTAA
GTATAAAATGACAAGTATAAGAAACGTTAAGCCTACAGCCGTAGAAGTT
CTAGATGAACAAAAAGGTAAAGATAAACAATTAACATTAATTACTTGTG
ATGATTACAATGAAGAGACAGGCGTTTGGGAAACCCGTAAAATCTTTGT
AGCTACAGAAGTCAAATAA
SEQ ID No.2: Ferritin_SEC 
ATGCCTATGGGATCTCTGCAGCCTCTGGCCACACTGTACCTGCTGGGAA
TGCTGGTGGCTAGCGTGCTGGCCGGCGGAGGCGGTTCTGGCGATATCAT
CAAGCTGCTGAACGAGCAAGTGAACAAAGAAATGCAATCCTCCAACC
TGTACATGAGCATGAGCAGCTGGTGTTACACCCACAGCCTTGATGGCGC
CGGACTGTTCCTGTTTGATCACGCCGCCGAGGAATACGAGCACGCCAA
GAAGCTGATCATCTTCCTGAACGAGAACAATGTGCCCGTGCAGCTGAC
CAGCATTAGCGCCCCAGAGCACAAGTTCGAGGGCCTGACACAAATCTT
TCAGAAGGCCTACGAACACGAGCAGCACATCTCCGAGAGCATCAACAA
CATCGTGGACCACGCCATTAAGTCCAAGGATCACGCCACCTTCAATTTT
CTGCAGTGGTACGTGGCCGAACAGCACGAGGAAGAAGTGCTGTTCAA
GGACATCCTGGACAAGATTGAGCTGATCGGCAACGAGAACCACGGCCT
GTATCTGGCCGACCAGTACGTGAAGGGAATCGCCAAGAGCCGGAAGTC
CGGCAGCCACCACCACCATCACCACTGATGA
SEQ ID No.3: CD19IgH (Blinatumomab_IgH_LPETGG) 
ATGGGATGGTCATGTATCATCCTTTTTCTAGTAGCAACTGCAaccggtgtaca
ttcccaggttcagctggttcagtctggcgccgaagtgaagaaacctggcagcagcgtgaaggtgtcctgcaaggcct
ctggctacgccttcagcagctactggatgaactgggtccgacaggcccctggacaaggcttggaatggatgggaca
gatctggcctggcgacagcgacaccaattacgcccagaaattccagggcagagtgaccatcaccgccgacgagtc
tacaagcaccgcctacatggaactgagcagcctgagaagcgaggacaccgccgtgtactactgtgccagacggga
aaccaccaccgtgggcagatattactacgccatggactactggggccagggcaccacagtgacagtctcttctgcgt
cgacCAAGGGCCCATCGGTCTTCCCCCTGGCACCCTCCTCCAAGAGCAC
CTCTGGGGGCACAGCGGCCCTGGGCTGCCTGGTCAAGGACTACTTCCC
CGAACCCGTGACGGTGTCGTGGAACTCAGGCGCCCTGACCAGCGGCGT
GCACACCTTCCCGGCTGTCCTACAGTCCTCAGGACTCTACTCCCTCAGC
AGCGTGGTGACCGTGCCCTCCAGCAGCTTGGGCACCCAGACCTACATC
TGCAACGTGAATCACAAGCCCAGCAACACCAAGGTGGACAAGAAAGT
TGAGCCCAAATCTTGTGACAAAACTCACACATGCCCACCGTGCCCAGC
ACCTGAACTCCTGGGGGGACCGTCAGTCTTCCTCTTCCCCCCAAAACC
CAAGGACACCCTCATGATCTCCCGGACCCCTGAGGTCACATGCGTGGT
GGTGGACGTGAGCCACGAAGACCCTGAGGTCAAGTTCAACTGGTACG
TGGACGGCGTGGAGGTGCATAATGCCAAGACAAAGCCGCGGGAGGAG
CAGTACAACGCCACGTACCGTGTGGTCAGCGTCCTCACCGTCCTGCAC
CAGGACTGGCTGAATGGCAAGGAGTACAAGTGCAAGGTCTCCAACAA
AGCCCTCCCAGCCCCCATCGCGGCAACCATCTCCAAAGCCAAAGGGC
AGCCCCGAGAACCACAGGTGTACACCCTGCCCCCATCCCGGGATGAG
CTGACCAAGAACCAGGTCAGCCTGACCTGCCTGGTCAAAGGCTTCTAT
CCCAGCGACATCGCCGTGGAGTGGGAGAGCAATGGGCAGCCGGAGAA
CAACTACAAGACCACGCCTCCCGTGCTGGACTCCGACGGCTCCTTCTT
CCTCTACAGCAAGCTCACCGTGGACAAGAGCAGGTGGCAGCAGGGGA
ACGTCTTCTCATGCTCCGTGATGCATGAGGCTCTGCACGCCCACTACA
CGCAGAAGAGCCTCTCCCTGTCTCCGGGTAAAAGCGGCGGCAGCGGC
CTGCCTGAGACCGGAGGATGA
SEQ ID No.4: CD19FabH (Blinatumomab_FabH_LPETGG) 
ATGGGATGGTCATGTATCATCCTTTTTCTAGTAGCAACTGCAaccggtgtacatt
cccaggttcagctggttcagtctggcgccgaagtgaagaaacctggcagcagcgtgaaggtgtcctgcaaggcctc
tggctacgccttcagcagctactggatgaactgggtccgacaggcccctggacaaggcttggaatggatgggacag
atctggcctggcgacagcgacaccaattacgcccagaaattccagggcagagtgaccatcaccgccgacgagtcta
caagcaccgcctacatggaactgagcagcctgagaagcgaggacaccgccgtgtactactgtgccagacgggaa
accaccaccgtgggcagatattactacgccatggactactggggccagggcaccacagtgacagtctcttctgcgtc
gacCAAGGGCCCATCGGTCTTCCCCCTGGCACCCTCCTCCAAGAGCACC
TCTGGGGGCACAGCGGCCCTGGGCTGCCTGGTCAAGGACTACTTCCCC
GAACCCGTGACGGTGTCGTGGAACTCAGGCGCCCTGACCAGCGGCGT
GCACACCTTCCCGGCTGTCCTACAGTCCTCAGGACTCTACTCCCTCAGC
AGCGTGGTGACCGTGCCCTCCAGCAGCTTGGGCACCCAGACCTACATC
TGCAACGTGAATCACAAGCCCAGCAACACCAAGGTGGACAAGAAAGT
TGAGCCCAAATCTTGTGACAAAAGCGGCGGCAGCGGCCTGCCTGAGAC
CGGAGGATGA
SEQ ID No.5: CD19IgK (Blinatumomab_IgK_LPETGG) 
ATGGGATGGTCATGTATCATCCTTTTTCTAGTAGCAACTGCAACCGGT
GTACATTCAGACATCCAGCTGACCCAGTCTCCAAGCTTTCTGAGCGCC
AGCGTGGGCGACAGAGTGACCATCACATGTAAAGCCAGCCAGAGCGT
GGACTACAGCGGCGACAGCTACCTGAACTGGTATCAGCAGAAGCCCG
GCAAGGCCCCTAAGCTGCTGATCTACGATGCCAGCAATCTGGTGTCCG
GCGTGCCCAGCAGATTTTCTGGCTCTGGAAGCGGCACCGAGTTCACCC
TGACCATATCTAGCCTGCAGCCTGAGGACTTCGCCACCTACTACTGCC
AGCAGAGCACCGAGAATCCCTGGACATTTGGCGGAGGCACCAAGCTG
GAAATCAAGCGTACGGTGGCTGCACCATCTGTCTTCATCTTCCCGCCA
TCTGATGAGCAGTTGAAATCTGGAACTGCCTCTGTTGTGTGCCTGCTG
AATAACTTCTACCCCAGAGAAGCCAAAGTGCAGTGGAAGGTGGACAA
CGCCCTGCAGAGCGGAAACAGCCAGGAAAGCGTGACAGAGCAGGATT
 
CCAAGGATTCCACATACAGCCTGAGCAGCACACTGACACTGTCCAAGG
CCGACTACGAGAAGCACAAGGTGTACGCCTGCGAAGTGACACACCAG
GGACTGTCCTCCCCTGTGACAAAGAGCTTCAACAGAGGAGAATGCTGA
SEQ ID No.6: CD20IgH (Rituximab_IgH_LPETGG) 
ATGGGATGGTCATGTATCATCCTTTTTCTAGTAGCAACTGCAACCGGT
GTACATTCCcaggtacaactgcagcagcctggggctgagctggtgaagcctggggcctcagtgaagatgtc
ctgcaaggcttctggctacacatttaccagttacaatatgcactgggtaaaacagacacctggtcggggcctggaatg
gattggagctatttatcccggaaatggtgatacttcctacaatcagaagttcaaaggcaaggccacattgactgcagac
aaatcctccagcacagcctacatgcagctcagcagcctgacatctgaggactctgcggtctattactgtgcaagatcg
acttactacggcggtgactggtacttcaatgtctggggcgcagggaccacggtcaccgtctctgcaGCGTCGA
C
SEQ ID No.7: CD20IgK (Rituximab_IgK_LPETGG) 
ATGGGATGGTCATGTATCATCCTTTTTCTAGTAGCAACTGCAACCGGT
GTACATTCAcaaattgttctctcccagtctccagcaatcctgtctgcatctccaggggagaaggtcacaatgac
ttgcagggccagctcaagtgtaagttacatccactggttccagcagaagccaggctcctcccccaaaccctggattta
tgccacatccaacctggcttctggagtccctgttcgcttcagtggcagtgggtctgggacttcttactctctcacaatca
gcagagtggaggctgaagatgctgccacttattactgccagcagtggactagtaacccacccacgttcggaggggg
gaccaagctggaaatcaaaCGTACG
SEQ ID No.8: CD22IgH (Inotuzumab_IgH_LPETGG) 
ATGGGATGGTCATGTATCATCCTTTTTCTAGTAGCAACTGCAACCGGTGT
ACATTCCGAAGTGCAGCTGGTTCAGTCTGGCGCCGAAGTGAAGAAACC
TGGCGCCTCTGTGAAGGTGTCCTGCAAGGCCAGCGGCTACCGcTTCAC
CAACTACTGGATaCACTGGGTCCGACAGGCCCCTGGACAAGGACTGGA
ATGGATCGGCGGCATCAACCCCGGCAACAACTACGCCACCTACCGGCG
GAAGTTTCAGGGCAGAGTGACCATGACCGCCGACACCAGCACAAGCA
CCGTGTACATGGAACTGAGCAGCCTGAGAAGCGAGGACACCGCCGTGT
ACTACTGCACCAGAGAAGGCTACGGCAACTACGGCGCTTGGTTTGCCT
 ATTGGGGCCAGGGCACACTGGTCACCGTTAGCTCTGCGTCGAC
SEQ ID No.9: CD22IgK (Inotuzumab_IgK_LPETGG) 
ATGGGATGGTCATGTATCATCCTTTTTCTAGTAGCAACTGCAACCGGTGT
ACATTCAGACGTGCAAGTGACACAGAGCCCTTCTAGCCTGTCTGCCAG
CGTGGGCGACAGAGTGACCATCACCTGTAGAAGCAGCCAGAGCCTGG
CCAACAGCTACGGCAATACCTTCCTGAGCTGGTATCTGCACAAGCCCGG
AAAGGCTCCCCAGCTGCTGATCTACGGCATCAGCAACAGATTCAGCGG
CGTGCCCGATAGGTTTAGCGGCTCTGGAAGCGGCACCGACTTCACCCT
GACCATATCTAGCCTGCAGCCTGAGGACTTCGCCACCTACTACTGTCTG
CAAGGCACCCACCAGCCTTACACCTTTGGCCAGGGCACCAAGGTGGAA
ATCAAGCGTACG
SEQ ID No.10: SRTA7_INT 
MHHHHHHSSGLVPRGSGMKETAAAKFERQHMDSPDLGTQAKPQIPKDKS
KVAGYIEIPDADIKEPVYPGPATREQLNRGVSFAKENASLDDQNISIAGHTF
IDRPNYQFTNLKAAKKGSMVYFKVGNETRKYKMTSIRNVKPTAVEVLDE
QKGKDKQLTLITCDDYNEETGVWETRKIFVATEVK
SEQ ID No.11: Ferritin_SEC 
MPMGSLQPLATLYLLGMLVASVLAGGGGSGDIIKLLNEQVNKEMQSSNLY
MSMSSWCYTHSLDGAGLFLFDHAAEEYEHAKKLIIFLNENNVPVQLTSIS
APEHKFEGLTQIFQKAYEHEQHISESINNIVDHAIKSKDHATFNFLQWYVAE
QHEEEVLFKDILDKIELIGNENHGLYLADQYVKGIAKSRKSGSHHHHHH
SEQ ID No.12: CD19IgH (Blinatumomab_IgH_LPETGG) 
MGWSCIILFLVATATGVHSQVQLVQSGAEVKKPGSSVKVSCKASGYAFSSY
WMNWVRQAPGQGLEWMGQIWPGDSDTNYAQKFQGRVTITADESTSTAY
MELSSLRSEDTAVYYCARRETTTVGRYYYAMDYWGQGTTVTVSSASTKG
PSVFPLAPSSKSTSGGTAALGCLVKDYFPEPVTVSWNSGALTSGVHTFPAV
 
LQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFN
WYVDGVEVHNAKTKPREEQYNATYRVVSVLTVLHQDWLNGKEYKCKV
SNKALPAPIAATISKAKGQPREPQVYTLPPSRDELTKNQVSLTCLVKGFYPS
DIAVEWESNGQPENNYKTTPPVLDSDGSFFLYSKLTVDKSRWQQGNVFSC
SVMHEALHAHYTQKSLSLSPGKSGGSGLPETGG
SEQ ID No.13: CD19FabH (Blinatumomab_FabH_LPETGG) 
MGWSCIILFLVATATGVHSQVQLVQSGAEVKKPGSSVKVSCKASGYAFSSY
WMNWVRQAPGQGLEWMGQIWPGDSDTNYAQKFQGRVTITADESTSTAY
MELSSLRSEDTAVYYCARRETTTVGRYYYAMDYWGQGTTVTVSSASTKG
PSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKSG
GSGLPETGG
SEQ ID No. 14: CD 19IgK (Blinatumomab_IgK_LPETGG) 
MGWSCIILFLVATATGVHSDIQLTQSPSFLSASVGDRVTITCKASQSVDYSG
DSYLNWYQQKPGKAPKLLIYDASNLVSGVPSRFSGSGSGTEFTLTISSLQP
EDFATYYCQQSTENPWTFGGGTKLEIKRTVAAPSVFIFPPSDEQLKSGTASV
VCLLNNFYPREAKVQWKVDNALQSGNSQESVTEQDSKDSTYSLSSTLTLS
KADYEKHKVYACEVTHQGLSSPVTKSFNRGEC
SEQ ID No.15: CD20IgH (Rituximab_IgH_LPETGG) 
MGWSCIILFLVATATGVHSQVQLQQPGAELVKPGASVKMSCKASGYTFTS
YNMHWVKQTPGRGLEWIGAIYPGNGDTSYNQKFKGKATLTADKSSSTAY
MQLSSLTSEDSAVYYCARSTYYGGDWYFNVWGAGTTVTVSAASTKGPSV
FPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWY
VDGVEVHNAKTKPREEQYNATYRVVSVLTVLHQDWLNGKEYKCKVSNK
 
ALPAPIAATISKAKGQPREPQVYTLPPSRDELTKNQVSLTCLVKGFYPSDIA
VEWESNGQPENNYKTTPPVLDSDGSFFLYSKLTVDKSRWQQGNVFSCSV
MHEALHAHYTQKSLSLSPGKSGGSGLPETGG
SEQ ID No.16: CD20IgK (Rituximab_IgK_LPETGG) 
MGWSCIILFLVATATGVHSQIVLSQSPAILSASPGEKVTMTCRASSSVSYIH
WFQQKPGSSPKPWIYATSNLASGVPVRFSGSGSGTSYSLTISRVEAEDAAT
YYCQQWTSNPPTFGGGTKLEIKRTVAAPSVFIFPPSDEQLKSGTASVVCLL
NNFYPREAKVQWKVDNALQSGNSQESVTEQDSKDSTYSLSSTLTLSKAD
YEKHKVYACEVTHQGLSSPVTKSFNRGEC
SEQ ID No.17: CD22IgH (Inotuzumab_IgH_LPETGG) 
MGWSCIILFLVATATGVHSEVQLVQSGAEVKKPGASVKVSCKASGYRFTN
YWIHWVRQAPGQGLEWIGGINPGNNYATYRRKFQGRVTMTADTSTSTVY
MELSSLRSEDTAVYYCTREGYGNYGAWFAYWGQGTLVTVSSASTKGPSV
FPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWY
VDGVEVHNAKTKPREEQYNATYRVVSVLTVLHQDWLNGKEYKCKVSNK
ALPAPIAATISKAKGQPREPQVYTLPPSRDELTKNQVSLTCLVKGFYPSDIA
VEWESNGQPENNYKTTPPVLDSDGSFFLYSKLTVDKSRWQQGNVFSCSV
MHEALHAHYTQKSLSLSPGKSGGSGLPETGG
SEQ ID No.18: CD22IgK (Inotuzumab_IgK_LPETGG) 
MGWSCIILFLVATATGVHSDVQVTQSPSSLSASVGDRVTITCRSSQSLANSY
GNTFLSWYLHKPGKAPQLLIYGISNRFSGVPDRFSGSGSGTDFTLTISSLQP
EDFATYYCLQGTHQPYTFGQGTKVEIKRTVAAPSVFIFPPSDEQLKSGTASV
VCLLNNFYPREAKVQWKVDNALQSGNSQESVTEQDSKDSTYSLSSTLTLS
KADYEKHKVYACEVTHQGLSSPVTKSFNRGEC
SEQ ID No.19: The Linker Sequence Formed by Sortase
   LPXTGGGG (where X may be any amino acid)
SEQ ID No.20: linked to the N-terminus of a ferritin monomer
   GGGG
SEQ ID No.21: linked to the C-terminus of a targeting protein molecule
   LPXTGG (where X may be any amino acid)

While the invention has been disclosed with reference to certain embodiments, it will be apparent that modifications and variations can be made without departing from the spirit and scope of the invention as disclosed herein and as provided by the appended claims. Furthermore, it is to be understood that while all examples in the disclosure illustrate embodiments of the invention, they are provided merely as non-limiting examples and thus should not be considered to limit the various aspects of the invention thereby illustrated. The invention is intended to have the full scope defined by this disclosure, the language of the following claims, and any equivalents thereof. Accordingly, the drawings and detailed description are to be regarded as illustrative rather than restrictive.

## Claims

1. A nano-delivery system, comprising a ferritin nanoparticle, a targeting protein, and an active molecule, wherein the targeting protein is conjugated onto the surface of the ferritin nanoparticle via the amino acid sequence set forth in SEQ ID NO. 19, and the active molecule is encapsulated within the ferritin nanoparticle.

2. The nano-delivery system according to claim 1, wherein the amino acid sequence of a ferritin in the ferritin nanoparticle is set forth in SEQ ID NO. 11.

3. The nano-delivery system according to claim 1 or 2, wherein the targeting protein is an antibody or an antigen-binding fragment thereof.

4. The nano-delivery system according to claim 3, wherein the antibody is an IgG antibody.

5. The nano-delivery system according to any one of claims 1 to 4, wherein the conjugation between the ferritin nanoparticle and the targeting protein is formed using Sortase A.

6. A method for preparing a nano-delivery system, comprising:
1) encapsulating an active molecule within a ferritin nanoparticle; and
2) conjugating the ferritin nanoparticle to a targeting protein using Sortase A.

7. The method according to claim 6, wherein steps 1) and 2) are performed in one reaction or in two separate reactions.

8. The method according to claim 6 or 7, further comprising a step of providing a ferritin having the amino acid sequence set forth in SEQ ID NO. 20 (GGGG) linked to its N-terminus and a targeting protein having the amino acid sequence set forth in SEQ ID NO. 21 (LPXTGG) linked to its C-terminus.

9. The method according to any one of claims 6 to 8, wherein the amino acid sequence of the ferritin is set forth in SEQ ID NO. 11.

10. The method according to any one of claims 6 to 9, wherein the targeting protein is an antibody or an antigen-binding fragment thereof, and the ferritin nanoparticle is connected to the C-terminus of a heavy chain of the antibody or antigen-binding fragment thereof using Sortase A.

11. A modified ferritin, having a sequence set forth in SEQ ID NO. 11.

12. An isolated polynucleotide, encoding a ferritin having a sequence set forth in SEQ ID NO. 11

13. Use of the modified ferritin according to claim 11 or the polynucleotide according to claim 12 in the preparation of a nano-delivery system.

14. Use of the nano-delivery system according to any one of claims 1 to 5 in the preparation of a medicament for treating a tumor.
